## Europäisches Patentamt
## European Patent Office
## Office européen des brevets

(19)

(11) Veröffentlichungsnummer: **0 152 949**
**B1**

(12) **EUROPÄISCHE PATENTSCHRIFT**

(45) Veröffentlichungstag der Patentschrift: **09.01.91**

(51) Int. Cl.⁵: **C 12 P 17/12**

(21) Anmeldenummer: **85101846.5**

(22) Anmeldetag: **20.02.85**

(54) **Verfahren zur Herstellung von 6-Hydroxynikotinsäure.**

(30) Priorität: **22.02.84 CH 840/84**

(43) Veröffentlichungstag der Anmeldung:
**28.08.85 Patentblatt 85/35**

(45) Bekanntmachung des Hinweises auf die
Patenterteilung:
**09.01.91 Patentblatt 91/02**

(84) Benannte Vertragsstaaten:
**AT BE CH DE FR GB IT LI NL SE**

(56) Entgegenhaltungen:
CHEMICAL ABSTRACTS, Band 61, Nr. 2, 20. Juli
1964, Spalte 2, Zusammenfassung Nr. 2208g,
Columbus, Ohio, US; J.C. ENSIGN et al.: "The
pathway of nicotinic acid oxidation by a Bacillus
species"

CHEMICAL ABSTRACTS, Band 47, Nr. 6, 25.
März 1953, Spalte 2, Zusammenfassung Nr.
2832h-i, Columbus, Ohio, US; D.E. HUGHES:
"6-Hydroxynicotinic acid as intermediate in
oxidation of nicotinic acid by Pseudomonas
fluorescens"

(73) Patentinhaber: **LONZA AG**
**Gampel/Wallis (CH)**

(72) Erfinder: **Kulla, Hans, Dr.**
**Wildi**
**Visp (Kanton Wallis) (CH)**
Erfinder: **Lehky, Pavel, Dr.**
**Dammweg 13**
**Naters (Kanton Wallis) (CH)**

(74) Vertreter: **Weinhold, Peter, Dr. et al**
**Patentanwälte Dr. V. Schmied-Kowarzik Dipl.-**
**Ing. G. Dannenberg Dr. P. Weinhold Dr. D. Gudel**
**Dipl.-Ing. S. Schubert Dr. P. Barz**
**Siegfriedstrasse 8**
**D-8000 München 40 (DE)**

(56) Entgegenhaltungen:
CHEMICAL ABSTRACTS, Band 54, Nr. 20, 25.
Oktober 1960, Spalte 1, Zusammenfassung Nr.
21307b-c, Columbus, Ohio, US; T. SUGAHARA:
"Microbial degradation of nicotinic acid and
nicotinamide"

EP 0 152 949 B1

**Beschreibung**

Die Erfindung betrifft ein Verfahren zur Herstellung von 6-Hydroxynikotinsäure auf biotechnischem Weg.

Mehrere Methoden zur Herstellung von 6-Hydroxynikotinsäure mittels organischer Synthesen sind bekannt: aus 2-Pyridon kann man die 6-Hydroxynikotinsäure durch die Kolbe-Schmidtische Carboxylierung von Hydroxyaromaten erhalten. Andere Synthesen geben von Aepfelsäure oder Isocinchomeronsäure aus [Briancourt et al., J.Chim.Ther. (1973), 8 (2) 226—32; Quarroz, CH-Anm. Nr. 7731/80].

Keine dieser bekannten Synthesen erlaubt eine einfache, billige und umweltfreundliche Herstellung von reiner 6-Hydroxynikotinsäure. Den Verfahren haftet der Nachteil an, dass die Umsetzung nicht quantitativ ist und unerwünschte Nebenprodukte die Reaktion begleiten. Die Nebenprodukte stellen eine Verunreinigung dar und müssen nach beendeter Reaktion aus dem Reaktionsprodukt entfernt werden.

Es ist auch bekannt, dass Mikroorganismen der Gattungen Bacillus, Pseudomonas, Clostridium und Mycobakterium auf Nikotinsäure wachsen und dass sie dieses Substrat als Kohlenstoff-, Stickstoff- und Energiequelle benutzen [Allinson M. J. C.: J.Biol. Chem. (1943) 147, 785; Behrman E. J., Stanier R. V, J.Biol. Chem (1957) 228, 923].

Die Nikotinsäure wird bei allen studierten Organismen im ersten Abbauschritt zu 6-Hydroxynikotinsäure oxidiert. Die 6-Hydroxynikotinsäure wird sofort und ohne bedeutende Anreicherung weiter umgewandelt, bei aeroben Organismen bis zu Wasser, Kohlendioxid und Ammoniak.

Nur nach Aufbrechen der Mikroorganismen war es möglich, die Nikotinsäure-Hydroxylase in mehr oder weniger reiner Form zu isolieren [Hunt A. L., Biochem. J. (1958) 72, 1—7]. Die Nikotinsäure-Hydroxylasen sind grosse Moleküle von etwa 400'000 Dalton. Sie enthalten Flavinkofaktoren, viele Metallatome (Fe, Mo), anorganischen Schwefel und in einigen Fällen sogar Selenium. Die Nikotinsäure-Hydroxylasen sind aktiv nur in Anwesenheit der geeigneten elektronübertragenden Systeme (z.B. Cytochrome, Flavine, $NADP^+$ und andere).

Es ist möglich, die Nikotinsäure-Hydroxylase aus Zell-Estrakten zu isolieren und die Enzympräparate zur Hydroxylierung der Nikotinsäure anzuwenden. Dies wurde gemacht und kleine Mengen von 6-Hydroxynikotinsäure wurden tatsächlich erhalten [Behrman u. Stanier, J.Biol.Chem (1957), 228 923]. Abgesehen von den hohen Enzym-Isolierungskosten und der Unstabilität der Nikotinsäure-Hydroxylase musste man noch für die Regenerierung von Kofaktoren und Elektronenübertragungs-Systeme sorgen.

Bei vielen Fermentationen und Enzymreaktionen sind die Produktkonzentrationen in der Reaktionslösung sehr tief und bei der Produktisolation müssen deshalb grosse Volumen aufgearbeitet werden. Das führt zu hohen Aufarbeitungs-, Anlage- und Abwasserkosten.

Eine ähnliche Situation herrscht auch bei der biotechnologischen Herstellung der 6-Hydroxynikotinsäure (CH-Anmeldung Nr. 825/84). Es ist zwar möglich, die enzymatische Hydroxylierung mit 0,1 Gew.%iger bis gesättigten Nikotinsäurelösungen durchzuführen. Die Stabilität des sich in den Zellen befindenden Enzyms ist bei höheren Substratkonzentrationen jedoch stark erniedrigt. Deshalb steigt auch der Enzymverbrauch und die Enzymkosten entsprechend.

Auf der anderen Seite ist das Wachstum von Mikroorganismen (Achromobacter) schon bei relativ tiefen Nikotinsäurekonzentrationen stark gehemmt. Die Enzymverluste könnten durch langsames Wachstum der Zellen während der Reaktion kompensiert werden. Das kann aber nur in verdünnten Nikotinsäurelösungen (0,1 bis 1,5%) geschehen.

Aus den erwähnten Tatsachen folgt, dass man die enzymatische Hydroxylierung der Nikotinsäure in verdünnten Lösungen durchführen soll; dies führt aber zu erhöhten Aufarbeitungskosten.

Aufgabe vorliegender Erfindung war es, diese Nachteile zu überwinden und ein Verfahren vorzuschlagen, das es ermöglicht, auf wirtschaftliche Weise aus Nikotinsäure die 6-Hydroxynikotinsäure in höchster Reinheit und Ausbeute herzustellen.

Erfindungsgemäss wird das mit einem Verfahren nach Patentanspruch 1 erreicht.

Der Bioreaktor wird zweckmässig mit einer 0,1 Gew.%igen bis gesättigten Lösung eines Magnesium- oder Bariumnikotinats gefüllt und die an Nikotinsäure- Hydroxylasen reichen Zellen zugegeben. Das während der Reaktion sich bildende Magnesium- oder Bariumhydroxynikotinat kristallisiert nach Erreichen der Sättigungsgrenze aus.

Die Reaktion wird bei Temperaturen von 10 bis 50°C, vorzugsweise bei 25 bis 35°C, durchgeführt.

Der pH-Wert wird durch Zusatz von Nikotinsäure konstant bei 5 bis 9 gehalten.

Eine bevorzugte Arbeitsweise der Erfindung ist ein kontinuierliches Verfahren, nach welchem eine 0,2 bis 10%ige Lösung eines Magnesium- oder Bariumsalzes der Nikotinsäure nach Massgabe der enzymatischen Hydroxylierung zur entsprechenden 6-Hydroxynikotinsäure in eine auf einem pH von 5 bis 9 gehaltene Suspension eines Achromobacter xylosoxydans einbringt und die anfallenden Magnesium- oder Bariumsalze der 6-Hydroxynikotinsäure laufend abtrennt.

Die schlechte Löslichkeit von Magnesium- oder Bariumhydroxynikotinat macht es möglich, das Produkt während der Reaktion als Magnesium- oder Barium-Salz selektiv ausfallen zu lassen, die Nikotinsäure bleibt in Lösung. Das Magnesium- oder Bariumhydroxynikotinat bildet feine Mikrokristalle, die sich durch Filtration oder Zentrifugation leicht abtrennen lassen. Aus dem Salz kann man durch ansäuren die reine 6-Hydroxynikotinsäure erhalten.

Die Zugabe des Eduktes in Form einer Lösung von Magnesium- oder Bariumnikotinat kann aufgrund der Leitfähigkeitsmessung mittels einer mit dem Leitfähigkeitsregler gekoppelten Pumpe automatisch erfolgen. Die Kristalle des Magnesium- oder Bariumhydroxynikotinats, die sich am Boden des Reaktionsgefässes oder in einem eingebauten Dekanteur absetzen, können periodisch oder kontinuierlich abgetrennt werden. Selbstverständlich kann man auch feste Nikotinsäure, zusammen oder parallel mit einer entsprechenden Menge des Magnesiumoxids, mittels eines Feststoffdosier-Gerätes in den Bioreaktor einführen.

So erhält man ein praktisch geschlossenes System, in welchem festes Edukt eingeführt wird und ein festes Produkt entnommen wird. Man kann in diesem System mit verdünnter Substratlösung arbeiten, d.h. unter Bedingungen, die eine gute Enzymstabilität garantieren, ohne dass Aufarbeitung und Abwasser-Probleme entstehen.

Die enzymatische Hydroxylierung wird mittels eines Mikroorganismus als Nikotinsäure-Hxdroxylase-Quelle ausgeführt.

Es wurde gefunden, dass Mikroorganismen der Gattungen Pseudomonas, Bacillus und Achromobacter die Herstellung der 6-Hydroxynikotinsäure mit Erfolg durchführen lässt: Zweckmässig wird der Achromobacter xylosoxydans DSM 2402 (type strain), Pseudomonas putida KB1 NCIB 10521, NCIB 8176 bzw. deren wirksame Mutanten, oder ein Bacillus-Stamm, der von Ensign und Rittenberg (J.Biol. Chem. *239*, (1964) 2285—2291) beschrieben wurde, angewendet.

Vorzugsweise wird der neue Stamm Achromobacter xylosoxydans DSM 2783 angewendet.

Name:
    Achromobacter xylosoxydans DSM Nr. 2783
Isoliert aus:
    Nikotinsäure-Mutterlauge

(A) Morphologie
    Kultivierung in Nutrient-Broth
        (1) Zellform: Stäbchen 2—3,5 μm lang, etwa 0,6 μm breit
        (2) Anordnung: einzeln
        (3) Motilität: stark beweglich; peritrich begeisselt
        (4) Endospore: strikt aerob
        (5) Gram: negativ
        (6) Oxidase: positiv
        (7) Catalase: positiv

Der Stamm stimmt in allen geprüften Merkmalen mit dem Typestrain von Achromobacter xylosoxydans (DSM 2402) überein (Ausnahme; Hydrolyse von Actamid).

Die genannten Stämme sind bei der Deutschen Sammlung von Mikroorganismen (DSM), Gesellschaft für Biotechnologische Forschung mbH., Griesebachstrasse 8, 4300 Göttingen, Bundesrepublik Deutschland, unter der Nr. DSM 2402 und DSM 2783 deponiert.

Der neue Stamm DSM 2783 wurde an oben genannter Hinterlegungsstelle an 18.11.1983 hinterlegt.

Die Stämme Pseudomonas putida NCIB 10521 und 8176 sind bei der National Collection of Industrial Bacteria, Torry Research Station, 135 Abbey Road, Aberdeen AB98DC, Scotland, erhältlich.

Die genannten Stämme wachsen mit Nikotinsäure als einzige Kohlenstoff-, Stickstoff- und Energiequelle.

Die Züchtung der genannten Mikroorganismen kann nach den für diese Art von Stämme bekannten Verfahren erfolgen.

Beispielsweise wird der Stamm DSM 2783 in einer verdünnten und sterilisierten Nikotinsäure-Lösung (0,05—0,5 Gew.%), die Phosphat-Puffer (50 mm) pH 7,0,

| Spurenelement (in mg/l) | |
|---|---|
| $CaCl_2 \cdot 2H_2O$ | 20 |
| $MnSO_4$ | 10 |
| $FeSO_4 \cdot 7H_2O$ | 5 |
| $CoCl_2 \cdot 6H_2O$ | 0,1 |
| $CuSO_4 \cdot 5H_2O1$ | 0,1 |
| $ZnSO_4 \cdot 7H_2O$ | 0,1 |
| $NaMoO_4 \cdot 2H_2O$ | 0,1 |

und, um das Wachstum zu beschleunigen, eine kleine Menge Hefeextrakt (Merck) (0,05 Gew.%) enthält, während 24 bis 48 Std. bei 30°C unter aeroben Bedingungen fermentiert.

Die gewachsene Biomasse (etwa 10 g Feuchtgewicht/l) ist reich an Nikotinsäure-Hydroxylase. Die Zellen werden abzentrifugiert und können sofort oder nach Lagerung bei −20°C direkt, d.h. ohne Enzymgewinnung oder Reinigung, für die Nikotinsäure-Hydroxylierung eingesetzt werden.

Für die Durchführung der Nikotinsäure-Hydroxylierung ist es wünschenswert, dass der Abbau der Nikotinsäure nicht über den ersten Schritt, nämlich die Hydroxylierung zur 6-Hydroxynikotinsäure, hinausgeht. In dieser Produktionsphase würde das Wachstum des Mikroorganismus auf Kosten der Ausbeute gehen.

Wie oben erwähnt, wächst der Stamm DSM 2783 gut in verdünnten Nikotinsäure-Lösungen (0,05—0,5%) und verbraucht die vorgelegte Nikotinsäure dabei vollständig.

Mit steigender Konzentration der Nikotinsäure wird das Zellwachstum gehemmt und oberhalb 2 Gew.% Nikotinsäure-Konzentration kann kein Wachstum mehr beobachtet werden. Die Aktivität der Nikotinsäure-Hydroxylase bleibt aber in den Zellen unverändert.

Der katabolische Abbau wird nach der Hydroxylierungsstufe unterbrochen. Deshalb sind die Neben- und Folgereaktionen eliminiert und die Reinheit und Ausbeute der 6-Hydroxynikotinsäure sind sehr hoch.

In den folgenden Beispielen wird die praktische Benutzung der Erfindung gezeigt.

Beispiel 1

Herstellung der Achromobacter xylosoxydans DSM 2783-Zellen

Eine Nährlösung, die 51,9 g $Na_2HPO_4 \cdot 2H_2O$, 20,0 g $KH_2PO_4$, 2,5 g Hefeextrakt und 10 g Nikotinsäure in 4750 ml Wasser enthielt, wurde in den Fermenter eingefüllt und 20 Min. bei 120°C sterilisiert. Nack Abkühlen auf 30°C wurde der Fermenter mit 500 ml der Starter-Kultur angeimpft und bei 30°C, pH 7,0, unter Begasung mit Luft 24 Std. fermentiert. Nach 25 Std. wurden 200 ml einer Lösung von 10 g Nikotinsäure und 2,5 g Hefeextrakt in Wasser steril zugegeben und die Fermentation weitergeführt. Nach 42 Std. wurde die Kultur geerntet und die Achromobacter-Zellen durch Zentrifugation (30 Min. bei 15'000 g) getrennt.

Man erhielt 38,3 g feuchte Biomasse.

In einem 7-1-Fermenter werden 2750 ml einer 2%igen Nikotinsäure-Lösung, die vorher durch Zugabe von festem Magnesiumoxid auf pH 7,1 gebracht wurde, eingefüllt und auf 30°C aufgewärmt. Die Hydroxylierung begann nach der Zugabe von 200 ml konzentrierter Suspension des Achromobacter xylosoxydans DSM 2783, (Endkonzentration $10^{10}$ Zellen/ml).

Nach Zugabe von Antischaummittel (z.B. Polypropylenglykol P-2000) wurde unter starkem Rühren belüftet. Der Fermenter wurde auf 30°C thermostatisiert. Der pH wurde mit Hilfe des pH-Stats und einer Lösung von Nikotinsäure als Korrekturmittel (Totalverbrauch 23 g) bei pH 7,0 gehalten. Die Substratkonzentration wurde mit dem Leitfähigkeitsmesser kontinuierlich gemessen. Dieser wurde über einen Regler mit einer peristaltischen Pumpe gekoppelt. Wenn die Substratkonzentration infolge der Bildung und des Ausfallens von Magnesiumhydroxynikotinat sank, wurde eine Vorratslösung von Magnesiumnikotinat (1M) so lange zugepumpt, bis die ursprüngliche Leitfähigkeit wieder erreicht wurde. So konnte man die Substrat-Konzentration automatisch kontrollieren.

Die Kristalle des Magnesiumhydroxynikotinats setzten sich am Boden des Fermentergefässes ab, sie wurden periodisch entnommen, abgenutscht und das Filtrat in den Fermenter zurückgeführt.

Die Anlage wurde 3 Tage lang kontinuierlich gefahren. Es wurden 3 l einmolare Magnesiumnikotinat-Lösung zugegeben. Nach beendeter Zugabe wurde der Reaktor noch während 5 Std gefahren, dann wurde er entleert. Die Suspension wurde abgenutscht. Es wurden 5,3 l Filtrat und 650 g feuchtes Magnesium- oder Bariumhydroxynikotinat erhalten.

Die gesamte Menge von Magnesiumhydroxynikotinat wurde in 2,5 l Wasser suspendiert und mit konzentrierter Salzsäure bis pH 1,2 angesäuert. Die ausgefallene 6-Hydroxynikotinsäure wurde abgenutscht. Die noch feuchten Kristalle wurde mit 400 ml Wasser gewaschen und getrocknet.

Man erhielt 468,8 g weisses, mikrokristallines Produkt, das laut HPLC-Analyse 98,8% 6-Hydroxynikotinsäure enthielt. Dies entsprach einer Ausbeute von 91,6%, auf die eingesetzte Nikotinsäure berechnet.

Beispiel 2

In einem 2,5-1-Fermenter wurde 1 l einer 3%igen wässrigen Suspension von Nikotinsäure eingefüllt und durch Zugabe von festem Bariumoxid auf pH 7,0 gebracht. Diese Substrat-Lösung wurde auf 30°C thermostatisiert. Nach Zugabe von Antischaummittel (Rhodorsil 70414) wurden 70 ml einer konzentrierten Suspension $OD^{550}$=90) von Achromobacter xylosoxydans DSM 2789 in den Fermenter eingeführt. Die Suspension wurde stark belüftet und gerührt. Der pH-Wert wurde durch Zugabe von fester Nikotinsäure auf 7,0 konstant gehalten. Die Zugabe von Substrat (einmolare Lösung von Bariumnikotinat) wurde wie in Beispiel 1 mittels Leitfähigkeitsmessung und Regelung automatisiert.

Nach 2 bis 3 Std erreichte die Konzentration des Bariumhydroxynikotinats die Sättigungsgrenze und das Produkt begann zu kristallisieren. Die Kristalle setzten sich am Boden des Fermentors ab und wurden periodisch abgenommen. Der Kristall-Brei wurde abgenutscht und das Filtrat zurückgeführt.

Die Anlage lief kontinuierlich 60 Std lang. Während der Hydroxylierung wurde 1 l einmolarer Nikotinsäure (Bariumsalz) in den Fermenter eingeführt. Zusätzlich wurden noch 6,8 g fest Nikotinsäure für die pH-Kontrolle verbraucht.

Der Fermenter wurde entleert und die an der Wand haftenden Kristalle von Bariumhydroxynikotinat abgekratzt. Die Suspension wurde abgenutscht. Man erhielt 1,9 1 Filtrat, das laut HPLC-Analyse 0,5% Nikotinsäure und 2,4% 6-Hydroxynikotinsäre enthielt.

Die gesamte Menge an feuchtem Bariumhydroxynikotinat wurde in 200 ml Wasser suspendiert. Dazu wurde konzentrierte Salzsäure zugegeben, bis der pH auf 1,2 sank. Nach 2-stündigem Rühren wurde die 6-Hydroxynikotinsäure abgenutscht, mit 100 ml Wasser gewaschen und im Vakuum bei 50°C getrocknet Man erhielt 118,9 g leicht gelbe 6-Hydroxynikotinsäure, die laut HPLC-Analyse einen Gehalt von 98,3% aufwies. Dies entsprach einer Ausbeute von 64,7%, bezogen auf die in das System eingeführte Nikotinsäure.

Wenn man die analytisch nachgewiesene Menge von im Filtrat zurückbleibende Bariumhydroxynikotinsäure mitrechnet, erhöhte sich die gesamte Ausbeute auf 89,9%.

Beispiel 3
(Schritt 1) Herstellung der Pseudomonas putida NCIB 8176-Biomasse.

Eine Nährlösung, die 52 g $Na_2HPO_4 \cdot 2H_2O$, 20 g $KH_2PO_4$, 2,5 g Hefeextrakt und 10 g Nikotinsäure in 4750 ml Wasser enthielt, wurde in einem 7 l-Chemap-Fermentor 20 Min. bei 120°C sterilisiert. Nach Abkühlen auf 30°C wurden 50 ml steriler Spurenelementlösung zugegeben, so dass die folgenden Endkonzentrationen (in ml/l) erreicht worden sind:

| | |
|---|---|
| $CaCl_{d2} \cdot 2H_2O$ | 20 |
| $MnSO_4$ | 10 |
| $FeSO_4 \cdot 7H_2O$ | 5 |
| $CoCl_2 \cdot 6H_2O$ | 0,1 |
| $CuSO_4 \cdot 5H_2O$ | 0,1 |
| $ZnSO_4 \cdot 7H_{d2}O$ | 0,1 |
| $NaMoO_4 \cdot 2H_2O$ | 0,1 |

Der Fermentor wurde mit 500 ml der Pseudomonas-Starter-Kultur angeimpft und bei 30°C, pH 7,0 unter Begasung mit Luft 24 Std. fermentiert. Nach 24 Std. wurden 200 ml einer sterilen Lösung von 10 g Nikotinsäure und 2,5 g Hefeextrakt in Wasser zugegeben und die Fermentation weitergeführt. Nach 38 Std. wurde die Zellmasse durch Zentrifugation (30 min. bei 10'000 g) abgetrennt.

Man erhielt 43,3 g feuchte Biomasse.

(Schritt 2) Hydroxylierung der Nikotinsäure.

In einem 3,5 l-Fermentor wurden 2 l einer 0,5%igen Nikotinsäure-Lösung mit Zugabe von festem Magnesiumoxid auf pH 7,0 gebracht und auf 30°C aufgewärmt.

Die Hydroxylierung begann nach der Zugabe von 20 g feuchter Pseudomonas putida NCIB 8176-Biomasse, die vorher in 100 ml Wasser aufgeschlemmt wurde. Gleichzeitig wurde Antischaummittel (Polypropylenglykol P-200, Fluka) zugegeben und in die Mischung Luft eingeführt, so dass die Konzentration des gelösten Sauerstofes im Bereich 3 bis 5 mg $O_2$/l blieb. Der pH wurde durch Zugabe von Nikotinsäure als Korrekturmittel (Totalverbrauch 15,2 g) bei pH 7,0 gehalten. Die Substratkonzentration wurde durch eine kontrollierte Zugabe von einmolarem Mg-Nikotinat mit Hilfe von Leitfähigkeitsmessung und Regelung konstant gehalten (siehe Beispiel 1).

Nach Erreichen der Sättigungskonzentration begann das Magnesiumsalz der 6-Hydroxynikotinsäure auszufallen. Die Kristalle, die sich am Boden des Fermentors absetzten, wurden periodisch entnommen und abgenutscht. Das jeweils anfallende Filtrat wurde für die Herstellung von neuer einmolarer Magnesiumnikotinat-Lösung benutzt. Die Kristalle wurden feuchte gelagert. Die Geschwindigkeit der Zugabe des Eduktes (einmolare Lösung von Magnesiumnikotinat) wurde gemessen und benutzt zur Bestimmung der totalen Enzymaktivität im Fermentor. Aktivitätsverluste wurden durch periodische Zugabe (1 mal pro Tag) von feuchter frischer Biomasse kompensiert.

Die Versuchsreihe dauerte 5 Tage. Während dieser Zeit wurden 1,6 l einmolares Magnesiumnikotinat in den Fermentor eingeleitet. Für die Ausbeutebestimmung wurde der Fermentor entleert und die an der Wand haftenden Kristalle abgekratzt. Die Kristalle wurden abgenutscht.

Das in der gesamte Versuchsreihe gewonnene Mg-Hydroxynikotinat wurde in 1,5 l Wasser suspendiert und durch Zugabe von konz. Salzsäure langsam auf pH 1,5 gebracht. Die Kristalle der 6-Hydroxynikotinsäure wurden abgenutscht, an der Nutsche mit 200 ml Wasser gewaschen und bei 60°C unter Vakuum getrocknet.

Man erhielt 365,0 g weisse Kristalle, die Laut HPLC-Analyse einen Gehalt von 99,3% aufwies. Dies entsprach einer Ausbeute von 92,4%, bezogen auf die in den Fermentor eingeführte Nikotinsäure.

## Patentansprüche

1. Verfahren zur Herstellung von 6-Hydroxynikotinsäure auf biotechnologischem Weg, dadurch gekennzeichnet, dass man Nikotinsäure in Gegenwart äquivalenter Mengen Magnesium- oder Bariumionen mit Hilfe Nikotinsäure-hydroxylierender Mikroorganismen enzymatisch hydroxyliert und das sich bildende und im Reaktionsgemisch ausfallende Magnesium- oder Bariumsalz der 6-Hydroxynikotinsäure abtrennt und aus diesen abgetrennten Salzen die 6-Hydroxynikotinsäure freisetzt.

2. Verfahren nach Patentanspruch 1, dadurch gekennzeichnet, dass man eine 0,1%ige bis gesättigte Lösung eines Magnesium- oder Bariumsalzes der Nikotinsäure verwendet, diese kontinuierlich nach Massgabe der enzymatischen Hydroxylierung zur entsprechenden 6-Hydroxynikotinsäure in eine auf einen pH von 5 bis 9 gehaltenen Suspension eines Achromobacter xylosoxydans einbringt und die ausfallenden Salze der 6-Hydroxynikotinsäure laufend abtrennt.

3. Verfahren nach Patentansprüchen 1 und 2, dadurch gekennzeichnet, dass man bei Temperaturen von 10 bis 50°C arbeitet.

4. Verfahren nach Patentansprüchen 1 bis 3, dadurch gekennzeichnet, dass man den pH durch Zusatz von Nikotinsäure konstant bei 5 bis 9 hält.

5. Verfahren nach Patentansprüchen 1 bis 4, dadurch gekennzeichnet, dass man die Reaktion kontinuierlich durchführt.

6. Verfahren nach Patentansprüchen 1 bis 5, dadurch gekennzeichnet, dass man einen Mikroorganismus der Gattungen Bacillus, Pseudomonas oder Achromobacter verwendet.

7. Verfahren nach Patentansprüchen 1 bis 6, dadurch gekennzeichnet, dass man einen Achromobacter xylosoxydans der Bezeichnung DSM 2783 oder eine wirksame Mutante von diesem verwendet.

## Revendications

1. Procédé de préparation de l'acide 6-hydroxynicotinique par voie biotechnologique, caractérisé en ce que l'on hydroxyle par voie enzymatique l'acide nicotinique en présence de quantités équivalentes d'ions magnésium ou baryum à l'aide de micro-organismes hydroxylant l'acide nicotinique et l'on isole le sel de magnésium ou de baryum de l'acide 6-hydroxynicotinique qui se forme et qui précipité dans le mélange réactionnel et on libère l'acide 6-hydroxynicotinique à partir de ces sels isolés.

2. Procédé selon la revendication 1, caractérisé en ce que l'on utilise une solution de concentration comprise entre 0,1% et la saturation d'un sel de magnésium ou de baryum de l'acide nicotinique, l'on introduit celle-ci en continu dans une suspension maintenue à un pH de 5 à 9 d'un Achromobacter xylosoxydans selon l'hydroxylation enzymatique en acide 6-hydroxynicotinique correspondant et l'on isole en continu les sels de l'acide 6-hydroxynicotinique qui précipitent.

3. Procédé selon les revendications 1 et 2, caractérisé en ce que l'on opère à des températures de 10 à 50°C.

4. Procédé selon les revendications 1 à 3, caractérisé en ce que l'on maintient constant le pH entre 5 et 9 par addition d'acide nicotinique.

5. Procédé selon les revendications 1 à 4, caractérisé en ce que l'on réalise la réaction en continu.

6. Procédé selon les revendications 1 à 5, caractérisé en ce que l'on utilise un micro-organisme des genres Bacillus, Pseudomonas ou Achromobacter.

7. Procédé selon les revendications 1 à 6, caractérisé en ce que l'on utilise un Achromobacter xylosoxydans dénommé DSM 2783 ou un mutant efficace de celui-ci.

**Claims**

1. Process for the preparation of 6-hydroxynicotinic acid by a biotechnological route, characterized in that nicotinic acid is enzymatically hydroxylated in the presence of equivalent amounts of magnesium or barium ions with the aid of nicotinic acid-hydroxylating microorganisms and the magnesium or barium salt of 6-hydroxynicotinic acid forming and precipitating from the reaction mixture is separated, and 6-hydroxynicotinic acid is liberated from said separated salts.

2. Process according to patent claim 1, characterized in that a solution of a magnesium or barium salt of nicotinic acid having a concentration of 0.1% up to saturation is used, said solution, depending on the enzymatic hydroxylation forming the corresponding 6-hydroxynicotinic acid, is continuously introduced into a suspension of Achromobacter xylosoxydans, maintained at pH 5 to 9, and the precipitating salt of 6-hydroxynicotinic acid are continuously separated.

3. Process according to patent claims 1 and 2, characterized in that one operates at temperatures of 10 to 50°C.

4. Process according to patent claims 1 to 3, characterized in that the pH is constantly maintained at 5 to 9 by the addition of nicotinic acid.

5. Process according to patent claims 1 to 4, characterized in that the reaction is carried out continuously.

6. Process according to patent claims 1 to 5, characterized in that a microorganism of the species Bacillus, Pseudomonas or Achromobacter is used.

7. Process according to patent claims 1 to 6, characterized in that the Achromobacter xylosoxydans DSM 2783 or an active mutant thereof is used.